(19) Europäisches Patentamt / European Patent Office / Office européen des brevets

(11) **EP 3 263 026 B1**

(12) **EUROPEAN PATENT SPECIFICATION**

(45) Date of publication and mention
of the grant of the patent:
**13.08.2025 Bulletin 2025/33**

(21) Application number: **16755111.8**

(22) Date of filing: **26.01.2016**

(51) International Patent Classification (IPC):
*A61B 10/00* (2006.01)     *A61B 5/00* (2006.01)
*A61B 5/369* (2021.01)     *A61B 5/377* (2021.01)

(52) Cooperative Patent Classification (CPC):
**A61B 5/4827; A61B 5/369; A61B 5/377;
A61B 10/00**

(86) International application number:
**PCT/JP2016/052145**

(87) International publication number:
**WO 2016/136361 (01.09.2016 Gazette 2016/35)**

(54) **PAIN MEASUREMENT DEVICE AND PAIN MEASUREMENT SYSTEM**

SCHMERZMESSVORRICHTUNG UND SCHMERZMESSSYSTEM

DISPOSITIF DE MESURE DE LA DOULEUR ET SYSTÈME DE MESURE DE LA DOULEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.02.2015 JP 2015034466**

(43) Date of publication of application:
**03.01.2018 Bulletin 2018/01**

(73) Proprietor: **Osaka University
Suita-shi, Osaka 565-0871 (JP)**

(72) Inventor: **NAKAE, Aya
Suita-shi, Osaka 565-0871 (JP)**

(74) Representative: **Ter Meer Steinmeister & Partner
Patentanwälte mbB
Nymphenburger Straße 4
80335 München (DE)**

(56) References cited:
WO-A1-03/000134      WO-A1-2013/140106
WO-A1-2014/059278    JP-A- 2006 263 113
JP-A- 2007 130 034    JP-A- 2010 523 226
JP-A- 2013 523 274    US-A1- 2008 249 430
US-A1- 2012 226 186   US-A1- 2014 066 739

**Description**

[Technical Field]

The present invention relates to a pain measurement device for measuring pain being experienced by a subject of measurement, based on brainwave data of the subject of measurement.

[Background Art]

While pain is fundamentally subjective, objective evaluation thereof is desired for treatment. Patients often suffer disadvantages by underestimating pain. In this regard, methods for objective measurement of pain have been proposed (see, for example, Patent Literature 1).

[Citation List]

[Patent Literature]

US patent application with publication number US 2008/0249430 discloses a method of detecting pain in a subject, comprising the steps of generating brain wave data based on brain wave activity of the subject and comparing the brain wave data to reference data to generate results data.
[PTL 1] Japanese National Phase PCT Laid-open Publication No. 2010-523226

[Summary of Invention]

[Technical Problem]

However, the above-described conventional techniques cannot correctly measure pain in some cases due to individual differences in subjects of measurement.
In this regard, the present invention provides a pain measurement device that can objectively and highly precisely measure pain being experienced bv a subject of measurement, as specified in the claims.

[Solution to Problem]

The pain measurement device according to one aspect of the present disclosure is a pain measurement device for measuring pain being experienced by a subject of measurement, comprising: a determination unit for determining a baseline stimulation amount corresponding to baseline pain in the subject of measurement based on a relationship between a pain level reported by the subject of measurement and a stimulation amount applied to the subject of measurement; and a measurement unit for measuring pain being experienced by the subject of measurement when brainwave data of a subject is measured by the comparing brainwave data of a subject measured from the subject of measurement, with reference brainwave data of the subject of measurement corresponding to the baseline stimulation amount.
With this configuration, the baseline stimulation amount corresponding to baseline pain in the subject of measurement can be determined, based on the relationship between the pain level reported by the subject of measurement and the stimulation amount applied to the subject of measurement. In addition, pain can be measured using reference brainwave data of the subject of measurement corresponding to the baseline stimulation amount. Thus, relative pain of the subject of measurement with respect to baseline pain can be measured using reference brainwave data suited to the individuality of the subject of measurement. As a result, pain being experienced by the subject of measurement can be objectively and highly precisely measured.
For example, in a relationship between the pain level and the stimulation amount, the determination unit may determine a representative value in a range of stimulation amounts, where a ratio of an increase in the pain level to an increase in the stimulation amount exceeds a predetermined threshold ratio, as the baseline stimulation amount.
With this configuration, the representative value in a range of stimulation amounts, where the ratio of an increase in the pain level to an increase in the stimulation amount exceeds the predetermined threshold ratio, can be determined as the baseline stimulation amount. Thus, the relationship between the pain level and the stimulation amount represented by a sigmoid curve can be utilized to attempt to normalize baseline pain. As a result, pain can be measured more objectively.
For example, the representative value may be at least one of a minimum value, a median value, and a maximum value.
With this configuration, at least one of the minimum value, the median value, and the maximum value can be used as a representative value.
For example, the measurement unit may calculate at least one characteristic value from each of the brainwave data of a subject and the reference brainwave data, and measure pain being experienced by the subject of measurement based on a result of comparison of at least one characteristic value calculated from the brainwave data of a subject, with at least one characteristic value calculated from the reference brainwave data.
With this configuration, pain being experienced by the subject of measurement can be measured, based on a characteristic value calculated from the brainwave data of a subject and a characteristic value calculated from the reference brainwave data. Thus, pain can be measured more effectively by comparing brainwave data of a subject and reference brainwave data.
For example, said at least one characteristic value may comprise a first characteristic value representing an amplitude of a brainwave's waveform induced by a stimulation.
With this configuration, a first characteristic value representing an amplitude of a brainwave's waveform can be used to compare the brainwave data of a subject with the reference brainwave data. Since the amplitude is dependent on the pain level, brainwave data can be more effectively compared, and pain can be measured with high precision.

For example, the measurement unit may determine that the subject of measurement has greater pain than the baseline pain, if a first evaluation value, indicating a relative size of a first characteristic value calculated from the brainwave data of a subject with respect to a first characteristic value calculated from the reference brainwave data, is greater than a first threshold value.

With this configuration, the subject of measurement can be determined to have greater pain than the baseline pain if the first evaluation value is greater than the first threshold value. Thus, pain can be measured with high precision by utilizing a characteristic of an amplitude being greater as pain intensifies.

For example, said at least one characteristic value may comprise a second characteristic value representing a latency in a brainwave's waveform induced by a stimulation.

With this configuration, the second characteristic value representing a latency in a brainwave's waveform can be used to compare the brainwave data of a subject with the reference brainwave data. Since a latency is dependent on the pain level, brainwave data can be compared more effectively, and pain can be measured with high precision.

For example, the measurement unit may determine that the subject of measurement has greater pain than the baseline pain, if a second evaluation value, indicating a relative size of a second characteristic value calculated from the brainwave data of a subject with respect to a second characteristic value calculated from the reference brainwave data, is less than a second threshold value.

With this configuration, it can be determined that the subject of measurement has greater pain than the baseline pain if the second evaluation value is greater than the second threshold value. Thus, pain can be measured with high precision by utilizing a characteristic of latency being shorter for greater pain.

For example, the pain measurement device may further comprise an estimation unit for estimating a second pain level to be reported by the subject of measurement and second brainwave data to be measured from the subject of measurement, when a stimulation at a second stimulation amount that is different from a plurality of first stimulation amounts is applied to the subject of measurement, based on a first pain level reported by the subject of measurement and first brainwave data measured from the subject of measurement when stimulations at the plurality of first stimulation amounts are individually applied to the subject of measurement; wherein the determination unit determines the reference stimulation amount based on a relationship between the first pain level and the second pain level, and the first stimulation amount and the second stimulation amount.

With this configuration, the pain level and brainwave data corresponding to a stimulation amount that is not actually applied to the subject of measurement can be estimated. Thus, the number of times a stimulation is applied to the subject of measurement can be reduced, and pain generated in the subject of measurement by the stimulation can be suppressed. In particular, significant pain generated in the subject of measurement by a large stimulation can be avoided by estimating the pain level and brainwave data corresponding to a large stimulation amount.

A pain measurement system according to one aspect of the present disclosure comprises: a pain measurement device for measuring pain being experienced by a subject of measurement, a stimulation device for applying stimulations at a plurality of stimulation amounts individually to the subject of measurement; and an electroencephalograph for (i) measuring each of brainwave data of the subject of measurement when the stimulations at the plurality of stimulation amounts are applied to the subject of measurement and (ii) measuring brainwave data of a subject used in measurement of pain of the subject of measurement, wherein the pain measurement device comprises a storing unit for storing brainwave data measured when the stimulations at the plurality of stimulation amounts are applied individually to the subject of measurement, along with a stimulation amount and a pain level reported by the subject of measurement, a determination unit for determining a baseline stimulation amount corresponding to baseline pain in the subject of measurement based on a relationship between the pain level reported by the subject of measurement and the stimulation amounts applied to the subject of measurement; and a measurement unit for measuring pain being experienced by the subject of measurement when the brainwave data of a subject is measured by comparing the brainwave data of a subject measured from the subject of measurement with reference brainwave data corresponding to the baseline stimulation amount.

With this configuration, an effect similar to that of the above-described pain measurement device can be achieved.

These generic or specific embodiments may be materialized with an integrated circuit, computer program, or a recording medium such as computer-readable CD-ROM, or with any combination of an integrated circuit, computer program, and a recording medium.

[Advantageous Effects of Invention]

According to one aspect of the present invention, pain being experienced by a subject of measurement can be objectively measured and measurement precision can be enhanced.

[Brief Description of Drawings]

[ Figure 1] Figure 1 is a block diagram showing a configuration of the pain measurement system in Embodiment 1.
[ Figure 2] Figure 2 is a flow chart showing the process of collecting brainwave data and pain levels in Embodiment 1.
[ Figure 3] Figure 3 is a flow chart showing the process of measuring pain in Embodiment 1
[ Figure 4] Figure 4 is a diagram showing one example of the relationship between pain levels and stimulation amounts in Embodiment 1.
[ Figure 5] Figure 5 is a diagram showing an example of a brainwave's waveform in Embodiment 1.
[ Figure 6] Figure 6 is a block diagram showing a configuration of the pain measurement system in Embodiment 2.
[ Figure 7] Figure 7 is a flow chart showing the process of measuring pain in Embodiment 2.

[Description of Embodiments]

Hereinafter, the embodiments are explained in detail while referring to the drawings.

Each of the embodiments explained below shows either a generic or a specific example. The numerical values, shapes, materials, constituent elements, arrangement position and connection form of the constituent elements, steps, the order of steps, and the like shown in the following embodiments are one example, which is not intended to limit the Claims. Further, constituent elements in the following embodiments that are not recited in an independent claim setting forth the superordinate concept are explained as an optional constituent element.

(Embodiment 1)[Configuration of pain measurement system]

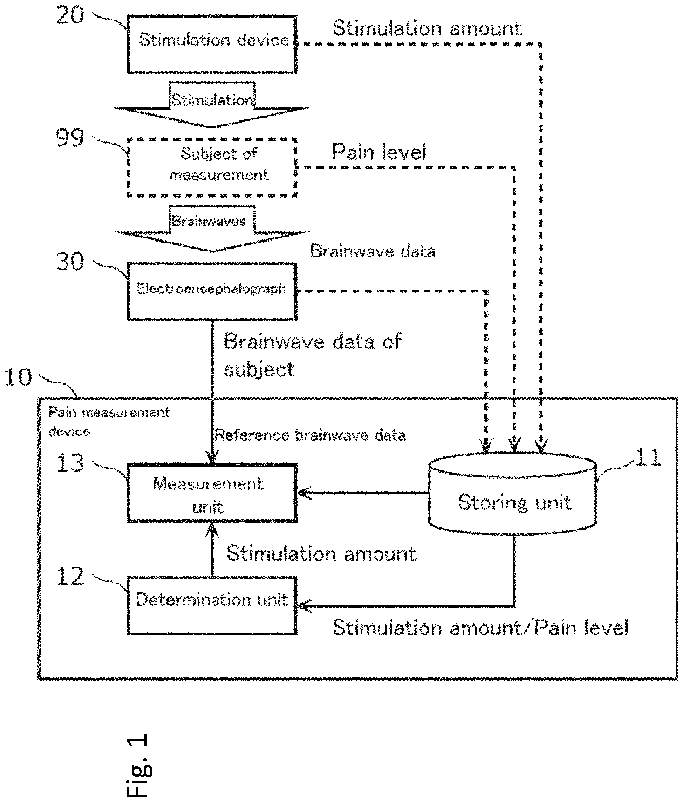

Figure 1 is a block diagram showing the configuration of the pain measurement system in Embodiment 1. The pain measurement system comprises a pain measurement device 10, a stimulation device 20, and an electroencephalograph 30.

The pain measurement device 10 measures pain being experienced by a subject of measurement 99. A subject of measurement is a living body in which pain induces a change in brainwaves. A subject of measurement does not need to be limited to humans.

The stimulation device 20 applies stimulations at a plurality of stimulation amounts individually to the subject of measurement 99. Specifically, the stimulation device 20 applies, for example, a plurality of stimulations in order while changing the stimulation amount to the subject of measurement 99.

In this regard, a stimulation is applied to the subject of measurement 99 from the outside of the subject of measurement 99 to induce pain of various levels to the subject of measurement 99. Specifically, a stimulation is, for example, electric stimulation or a thermal stimulation.

The subject of measurement 99 subjectively reports the pain level indicating the degree of pain generated by a stimulation applied by the stimulation device 20. For example, the subject of measurement 99 reports the pain level in a visual analog scale (VAS) when a stimulation is applied. VAS is a method of reporting a pain level by indicating which position the current pain level is on a 10 cm straight line representing the pain level from 0 to 100.

The electroencephalograph 30 measures electric activity generated in the brain of the subject of measurement 99 with an electrode on the scalp. In addition, the electroencephalograph 30 outputs results of measurement, brainwave data. Specifically, the electroencephalograph 30 measures each brainwave data when stimulations at a plurality of stimulation amounts are applied to the subject of measurement 99 by the stimulation device 20. Furthermore, the electroencephalograph 30 measures brainwave data of a subject used in the measurement of pain of the subject of measurement 99.

The stimulation amount of a stimulation applied to the subject of measurement 99 by the stimulation device 20, the pain level reported by the subject of measurement 99, and brainwave data that is output from the electroencephalograph 30 are input into the pain measurement device 10 and are used for obtaining reference brainwave data.

[Functional configuration of pain measurement device]

Next, the detailed configuration of the pain measurement device 10 is explained. As shown in Figure 1 , the pain measurement device 10 comprises a storing unit 11, a determination unit 12, and a measurement unit 13.

The storing unit 11 is, for example, a hard drive or a semiconductor memory. The storing unit 11 stores a stimulation amount of a stimulation applied to the subject of measurement 99 by the stimulation device 20, the pain level reported by the subject of measurement 99, and brainwave data output from the electroencephalograph 30.

The determination unit 12 is materialized by, for example, a processor and a memory. The determination unit 12 determines a baseline stimulation amount corresponding to baseline pain in the subject of measurement 99, based on the relationship between the pain level reported by the subject of measurement 99 and the stimulation amount applied to the subject of measurement 99. For example, the determination unit 12 determines a representative value (e.g., the minimum value, the median value, the maximum value, or the like) in a range where a pain level increases with an increase in the stimulation amount in the relationship between the pain level reported by the subject of measurement 99 and the stimulation amount applied to the subject of measurement 99 as a baseline stimulation amount. The determination of the baseline stimulation amount is discussed in detail below.

The measurement unit 13 measures the pain being experienced by the subject of measurement 99 when brainwave data of a subject is measured by comparing the brainwave data of a subject measured from the subject of measurement 99 with the reference electroencephalograph data. Electroencephalograph data of a subject is electroencephalograph data measured from the subject of measurement 99 for measuring pain being experienced by the subject of measurement 99. Further, reference electroencephalograph data is electroencephalograph data referred to for measuring pain from electroencephalograph data of a subject. In this Embodiment, reference electroencephalograph data is electroencephalograph data measured from the subject of measurement 99 when a stimulation at a baseline stimulation amount is applied to the subject of measurement 99.

For example, at least one characteristic value calculated from each of brainwave data of a subject and reference electroencephalograph data is used for comparison of the brainwave data of a subject with the reference electroencephalograph data. The measurement of pain by comparing brainwave data of a subject with reference brainwave data is discussed in detail below.

[Operation of pain measurement system]

Next, the operation of a pain measurement system with the above configuration is explained.

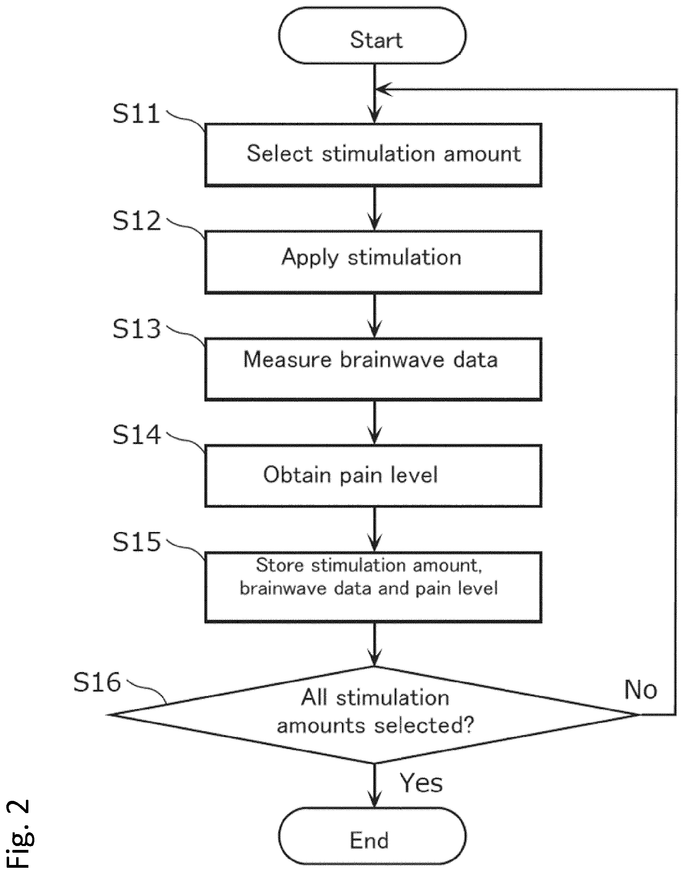

First, Figure 2 is used to explain the process of collecting brainwave data and pain levels. Figure 2 is a flow chart showing the process of collecting brainwave data and pain levels in Embodiment 1.

In the stimulation device 20, one stimulation amount that has not been selected is selected from a plurality of stimulation amounts (S11). For example, one stimulation amount that has not been selected is selected from electric stimulation amounts of 20 $\mu$A, 40 $\mu$A, 60 $\mu$A, 80 $\mu$A, and 100 $\mu$A.

Next, the stimulation device 20 applies a stimulation at the selected stimulation amount to the subject of measurement 99 (S12). When a stimulation is applied in step S12, the electroencephalograph 30 measures brainwave data from the subject of measurement 99 (S13). Furthermore, the pain level reported by the subject of measurement 99 in response to the stimulation applied in step S12 is obtained (S14).

The stimulation amount selected in step S11, the brainwave data measured in step S13, and the pain level obtained in step S14 are stored in the storing unit 11 (S15).

If all of the plurality of stimulation amounts have already been selected (Yes in S16), the process ends. If one of the plurality of stimulation amounts has not been selected (No in S16), the process returns to step S11.

As disclosed above, stimulations at a plurality of stimulation amounts are applied to the subject of measurement 99 in order, and brainwave data and pain level corresponding to each stimulation amount are stored in the storing unit 11.

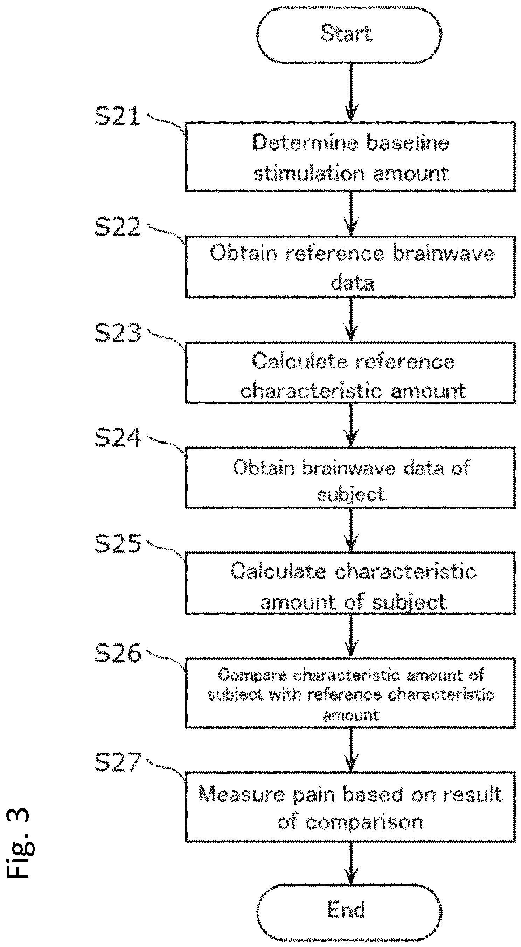

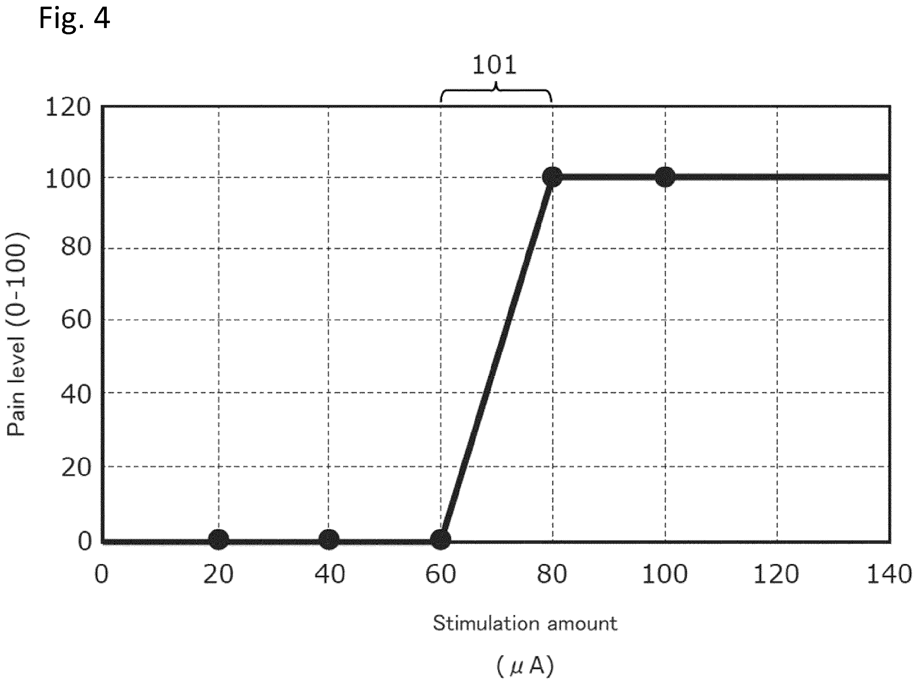

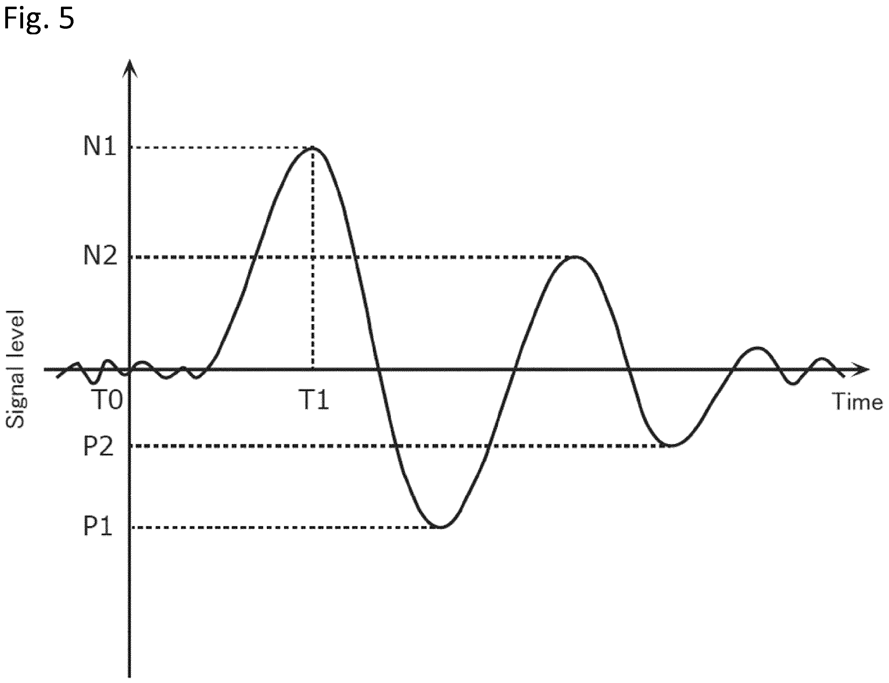

Next, Figures 3-5 are used to explain the process of measuring pain. Figure 3 is a flow chart showing the process of measuring pain in Embodiment 1. Figure 4 is a diagram showing one example of the relationship between the pain level and stimulation amount in Embodiment 1. Figure 5 is a diagram showing an example of a brainwave's waveform in Embodiment 1.

The determination unit 12 determines a baseline stimulation amount corresponding to baseline pain in the subject of measurement 99, based on the relationship between the pain level reported by the subject of measurement 99 and the stimulation amount applied to the subject of measurement 99 (S21).

As shown in Figure 4, the relationship between pain level and stimulation amount is represented by a sigmoid (S-shaped) curve. However, the shape of a sigmoid curve (e.g., maximum value, minimum value, and the like) is different for each subject of measurement. Thus, pain levels are normalized by utilizing a characteristic point (e.g., inflection point or the like) in a sigmoid curve in this Embodiment. Specifically, the determination unit 12 determines a representative value (e.g., the minimum value, the median value, the maximum value, or the like) in a range of stimulation amounts, where the pain level increases with an increase in the stimulation amount in a relationship between the pain level and the stimulation amount as a baseline stimulation amount.

The range of stimulation amounts where the pain level increases with an increase in the stimulation amount is determined by comparing the ratio of increase in the pain level to an increase in the stimulation amount with a predetermined threshold ratio. That is, the range of stimulation amounts, where the pain level increases with an increase in the stimulation amount, is a range where a ratio of increase in the pain level to an increase in the stimulation amount exceeds a predetermined threshold ratio. A predetermined threshold ratio may be experimentally or empirically determined.

The minimum value of a range of stimulation amounts, where the pain level increases with an increase in the stimulation amount, corresponds to a stimulation amount at which an increase in pain level starts. That is, the minimum value in said range is the maximum stimulation amount among stimulation amounts where the pain level does not decrease any more even with a decrease in the stimulation amount.

The maximum value in a range of stimulation amounts where the pain level increases with an increase in the stimulation amount corresponds to a stimulation amount at which the pain level stops increasing. That is, the maximum value in said range is the minimum stimulation amount among stimulation amounts where the pain level does not increase any more even with an increase in the stimulation amount.

In Figure 4 , the pain level increases with an increase in the stimulation amount in the range 101. In this regard, the determination unit 12 determines, for example, the minimum value (60 μA) in the range 101 as the baseline stimulation amount. As another example, the determination unit 12 may determine the median value (70 μA) in the range 101 as the baseline stimulation amount. As another example, the determination unit 12 may determine the maximum value (80 μA) in the range 101 as the baseline stimulation amount. As another example, the determination unit 12 may determine all or any two of the minimum value, the median value, and the maximum value in the range 101 as the baseline stimulation amounts.

Next, the measurement unit 13 obtains reference brainwave data from the storing unit 11 (S22). That is, the measurement unit 13 obtains brainwave data measured from the subject of measurement 99 when a stimulation at the baseline stimulation amount is applied to the subject of measurement 99 from the storing unit 11 as reference brainwave data.

Subsequently, the measurement unit 13 calculates a reference characteristic value from the reference brainwave data (S23). Specifically, the measurement unit 13 calculates reference characteristic values comprising a first characteristic value and a second characteristic value from the reference brainwave data.

In this Embodiment, the first characteristic value represents an amplitude of a brainwave's waveform induced by a stimulation. Specifically, the first characteristic value is, for example, the difference between the maximum peak value and the minimum peak value (i.e., peak to peak value). For example in Figure 5 , the measurement unit 13 calculates the maximum difference (N1-P1) among three differences (N1-P1, N2-P2, and N1-P2) as the first characteristic value.

In this Embodiment, the second characteristic value represents a latency in a brainwave's waveform induced by a stimulation. A latency is the time period between application of a stimulation and a reaction. Specifically, the second characteristic value is, for example, a time period between application of a stimulation and a first peak of a brainwave's waveform induced by the stimulation. For example in Figure 5 , the measurement unit 13 calculates the time period (T1-T0) from time T0 at which a stimulation is applied to time T1 at which the first peak appears as the second characteristic value.

Next, the measurement unit 13 obtains brainwave data of a subject (S24). That is, the measurement unit 13 obtains brainwave data measured from the subject of measurement 99 as brainwave data of a subject for measuring the pain being experienced by the subject of measurement 99.

Subsequently, the measurement unit 13 calculates brainwave data of a subject from a characteristic value of a subject (S25). Specifically, the measurement unit 13 calculates characteristic values of a subject comprising a first characteristic value and a second characteristic value from brainwave data of a subject as in step S23.

The measurement unit 13 then compares the characteristic value of a subject with the reference characteristic value (S26). Specifically, the measurement unit 13 compares the first characteristic value comprised in the characteristic value of a subject, with the first characteristic value comprised in the reference characteristic value. In this Embodiment, the measurement unit 13 calculates a first evaluation value indicating the relative size of the first characteristic value comprised in the characteristic value of a subject with respect to the first characteristic value comprised in the reference characteristic value, as a result of comparison of the first characteristic values. The first evaluation value is calculated using, for example, the following equation (1).

$$E1 = 100 \times Ax - Ar / Ar \ldots (1)$$

Wherein E1 represents the first evaluation value; Ax represents the first characteristic value comprised in the characteristic value of a subject; and Ar represents the first characteristic value comprised in the reference characteristic value.

Furthermore, the measurement unit 13 compares the second characteristic value comprised in the characteristic value of a subject, with the second characteristic value comprised in the reference characteristic value. In this Embodiment, the measurement unit 13 calculates a second evaluation value, indicating the relative size of the second characteristic value comprised in the characteristic value of a subject with respect to the second characteristic value comprised in the reference characteristic value, as a result of comparison of the second characteristic values. The second evaluation value is calculated using, for example, the following equation (2) .

$$E2 = Bx - Br \ldots (2)$$

Wherein E2 represents the second evaluation value; Bx represents the second characteristic value comprised in the characteristic value of a subject; and Br represents the second characteristic value comprised in the reference characteristic value.

In this manner, the measurement unit 13 calculates the first evaluation value and the second evaluation value as a result of comparison of the characteristic value of a subject with the reference characteristic value of a subject.

Next, the measurement unit 13 measures pain being experienced by the subject of measurement 99, based on the result of comparison of the characteristic value of a subject with the reference characteristic value of a subject (S27). In this Embodiment, the measurement unit 13 determines that the subject of measurement 99 has greater pain than pain corresponding to the baseline stimulation amount if the first evaluation value and the second evaluation value meet a predetermined condition.

Specifically, the measurement unit 13 determines that the subject of measurement 99 has greater pain than pain corresponding to the baseline stimulation amount if, for example, the first evaluation value is greater than the first threshold value and the second evaluation value is less than the second threshold value. In this case, the predetermined condition is represented by the following (3).

$$E1 > Th\,1 \text{ and } E2 < Th2 \dots (3)$$

Wherein Th1 is the first threshold value; and Th2 is the second threshold value.

For instance, if the minimum value in the range 101 is used as the baseline stimulation amount, the measurement unit 13 determines that "there is pain" if condition (3) is met. If condition (3) is not met, it determines that "there is no pain". Further, if the median value in the range 101 is used as the baseline stimulation amount, the measurement unit 13 determines that "there is medium level or greater pain" if condition (3) is met, and determines that "there is no pain" or "there is low level of pain" if condition (3) is not met. Further, if the maximum value in the range 101 is used as the baseline stimulation amount, the measurement unit 13 determines that "there is high level of pain" if condition (3) is met, and determines that "there is no pain" or "there is a medium level or less pain" if condition (3) is not met. The measurement unit 13 can distinguish the degree of pain being experienced by the subject of measurement 99 into one of "none", "low level", "medium level", and "high level" by using these three baseline stimulation amounts in combination.

[Effect]

In view of the above, with the pain measurement device 10 according to this Embodiment, the determination unit 12 can determine the baseline stimulation amount corresponding to baseline pain in the subject of measurement 99, based on the relationship between the pain level reported by the subject of measurement 99 and the stimulation amount applied to the subject of measurement 99. In addition, the measurement unit 13 can measure pain using reference brainwave data of the subject of measurement 99 corresponding to the baseline stimulation amount. Thus, the pain measurement device 10 can measure relative pain of the subject of measurement 99 with respect to the baseline pain using reference brainwave data suited to the individuality of the subject of measurement 99. As a result, the pain measurement device 10 can objectively and highly precisely measure pain being experienced by the subject of measurement 99.

With the pain measurement device 10 according to this embodiment, the determination unit 12 can determine the representative value in the range of stimulation amounts, where the pain level increases with an increase in the stimulation amount, as the baseline stimulation amount. Thus, the determination unit 12 can achieve normalization of the baseline pain by utilizing the relationship between the pain level and a plurality of stimulation amounts represented by a sigmoid curve. As a result, the pain measurement device 10 can measure pain more objectively.

With the pain measurement device 10 according to this embodiment, the measurement unit 13 can measure pain being experienced by a subject of measurement, based on a result of comparison of the characteristic value calculated from the brainwave data of a subject and the characteristic value calculated from the reference brainwave data. Thus, the measurement unit 13 can compare brainwave data of a subject with reference brainwave data more effectively.

With the pain measurement device 10 according to this embodiment, the measurement unit 13 can compare brainwave data of a subject with reference brainwave data using the first characteristic value representing an amplitude of a brainwave's waveform. Since amplitude is dependent on pain level, the measurement unit 13 can compare brainwave data more effectively and measure pain with high precision.

With the pain measurement device 10 according to this embodiment, the measurement unit 13 can determine that a subject of measurement has greater pain than baseline pain if the first evaluation value is greater than the first threshold value. Thus, the measurement unit 13 can measure pain with high precision by utilizing a characteristic of an amplitude being greater for greater pain.

The inventor actually conducted an experiment using "10" as the first threshold value on 15 subjects. As a result, it was possible to correctly distinguish the presence/absence of pain in 11 subjects.

With the pain measurement device 10 according to this embodiment, the measurement unit 13 can compare brainwave data of a subject with reference brainwave data using the second characteristic value representing a latency in a brainwave's waveform. Since a latency is dependent on the pain level, the measurement unit 13 can compare brainwave data more effectively and measure pain with high precision.

With the pain measurement device 10 according to this embodiment, the measurement unit 13 can determine that a subject of measurement has greater pain than baseline pain if the second evaluation value is greater than the second threshold value. Thus, the measurement unit 13 can measure pain more precisely by utilizing a characteristic of latency being shorter for greater pain.

The inventor actually conducted an experiment using "0" as the second threshold value on 15 subjects. As a result, it was possible to correctly distinguish the presence/absence of pain in 7 subjects.

The inventor actually conducted a further experiment using "10" as the first threshold value and "0" as the second threshold value on 15 subjects. As a result, it was possible to correctly distinguish the presence/absence of pain in 12 subjects.

(Embodiment 2)

Next, Embodiment 2 is explained. This embodiment is different from the above-described Embodiment 1 in that a pain level and brainwave data corresponding to a stimulation amount that is not actually applied to a subject of measurement are estimated, and the result of estimation is used to determine a baseline stimulation amount. This embodiment is explained hereinafter mainly with respect to the above-described difference from Embodiment 1.

[Configuration of pain measurement system]

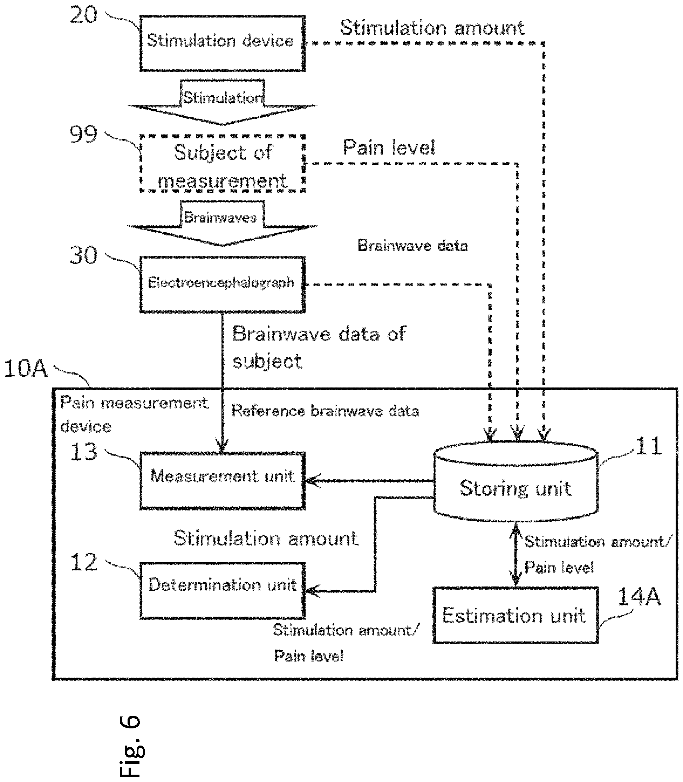

Figure 6 is a block diagram showing a configuration of the pain measurement system in Embodiment 2. In Figure 6 , constituent elements that are substantially the same as Figure 1 are assigned with the same symbol, and explanation thereof is omitted when appropriate. The pain measurement system according to this embodiment comprises a pain measurement device 10A, a stimulation device 20, and an electroencephalograph 30.

The pain measurement device 10A measures pain being experienced by a subject of measurement 99. The pain measurement device 10A comprises a storing unit 11, determination unit 12, a measurement unit 13, and an estimation unit 14A.

The estimation unit 14A estimates a pain level and brainwave data corresponding to a stimulation amount that is not actually applied to the subject of measure 99, based on a pain level actually reported by the subject of measurement 99 and brainwave data measured from the subject of measurement 99. That is, the estimation unit 14A estimates a second pain level to be reported by the subject of measurement 99 and second brainwave data to be measured from the subject of measurement 99 when stimulations at second stimulation amounts that are different from a plurality of first stimulation amounts is applied to the subject of measurement 99, based on the first pain level reported by the subject of measurement 99 and first brainwave data measured from the subject of measurement 99 when stimulations at the plurality of first stimulation amounts are individually applied to the subject of measurement 99. In this regard, the estimated pain level and brainwave data are stored in the storing unit 11 with the stimulation amounts. The brainwave data estimated by the estimation unit 14A does not need to be a brainwave's waveform and may be a characteristic value of brainwaves (e.g., amplitude, latency, or the like).

A method of estimating a pain level and brainwave data by the estimation unit 14A may be any method. Such a method is not particularly limited. For example, the method of estimation may use regression analysis or machine learning. For example, the estimation unit 14A may estimate a hypothetical pain level and brainwave data of the subject of measurement 99 upon application of a stimulation at a stimulation amount that is not actually applied to the subject of measurement 99, by applying regression analysis on a pain level reported by the subject of measurement 99 and brainwave data measured from the subject of measurement 99, when stimulations at a plurality of stimulation amounts are applied individually to the subject of measurement 99. As another example, the estimation unit 14A may estimate a pain level and brainwave data for a stimulation amount that is not actually applied to the subject of measurement 99, by using a model constructed by machine learning using a pain level and brainwave data obtained by applying a stimulation to a living body that is different from the subject of measurement 99 as training data.

[Operation of pain measurement system]

Next, the operation of a pain measurement system configured in the above manner is explained. The process of collecting brainwave data and pain levels is substantially the same as Embodiment 1 except for the number of stimulations applied. Thus, illustration thereof is omitted.

In this embodiment, the subject of measurement 99 is only applied with a stimulation in a small stimulation amount, in order to suppress pain caused by the stimulation applied to the subject of measurement 99. For example, the stimulation device 20 applies an electric stimulation of 70 μA or less to the subject of measurement 99. As a result, the pain level reported by the subject of measurement 99 and brainwave data measured from the subject of measurement 99 when electric stimulations of 70 μA or less are individually applied are stored in the storing unit 11 with stimulation amounts.

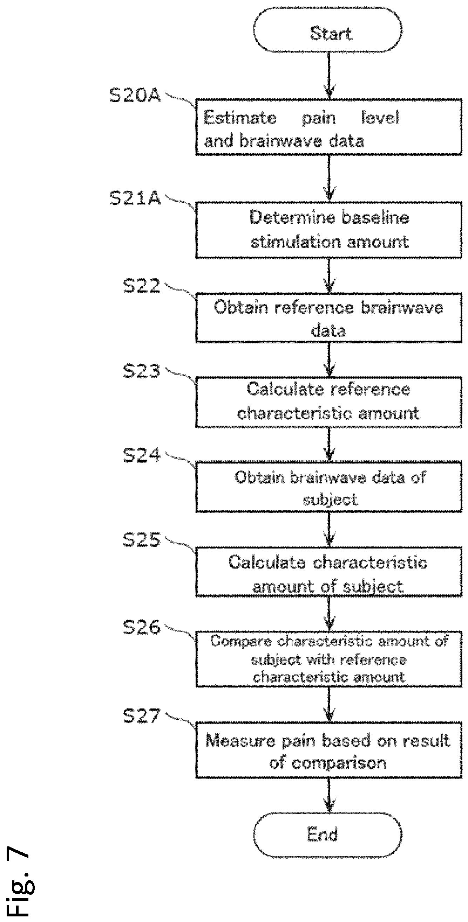

Figure 7 is a flow chart showing the process of measuring pain in Embodiment 2. In Figure 7 , processing that is substantially the same as Figure 4 is assigned with the same symbol, and explanation thereof is omitted when appropriate.

In this embodiment, a pain level and brainwave data for a stimulation amount that is not actually applied to the subject of measurement 99 are estimated before determining a baseline stimulation amount (S20A). Specifically, the estimation unit 14A estimates a pain level to be reported by the subject of measurement 99 (i.e., second pain level) and brainwave data to be measured from the subject of measurement 99 (i.e., second brainwave data), when a stimulation of unknown amount is applied to the subject of measurement 99, based on the pain level (i.e., first pain level) and brainwave data (i.e., first brainwave data) stored in the storing unit 11 in step S15.

For example, the estimation unit 14A estimates the pain level to be reported by the subject of measurement 99 and brainwave data to be measured from the subject of measurement 99 when an electric stimulation greater than 70 μA is applied to the subject of measurement 99 using the relationship between the pain level reported by the subject of measurement 99 when an electric stimulation of 70 μA or less is actually applied to the subject of smeasurement 99 and the amount of electric stimulation. It should be noted that the relationship between the size of electric stimulation actually applied and the size of electric stimulation at which pain level or the like is estimated is not limited thereto. For example, an electric stimulation greater than 70 μA may be actually applied to the subject of measurement 99, and the pain level or the like corresponding to an electric stimulation of 70 μA or less may be estimated. As another example, an electric stimulation of 60 μA to 80 μA may be actually applied to the subject of measurement 99, and a pain level or the like corresponding to an electric stimulation less than 60 μA or 80 μA may be estimated.

Subsequently, the determination unit 12 determines a baseline stimulation amount corresponding to baseline pain in the subject of measurement 99, based on the relationship between the stimulation amount and the pain level (S21A) stored in the storing unit 11. That is, the determination unit 12 determines a baseline stimulation amount using both the actually reported pain level and the estimated pain level.

[Effect]

As discussed above, with the pain measurement device 10A according to this embodiment, the estimation unit 14A can estimate a pain level and brainwave data corresponding to a stimulation amount that is not actually applied to the subject of measurement 99. Thus, a pain measurement system can reduce the number of stimulations applied to the subject of measurement 99 and suppress pain generated in the subject of measurement 99 by a stimulation. In particular, significant pain generated in the subject of measurement 99 with a large stimulation can be avoided by estimating a pain level and brainwave data corresponding to a large stimulation amount.

(Other embodiments)

The pain measurement devices according to one or more aspects of the present invention have been explained above based on embodiments. The present invention, however, is not limited to these embodiments.

For example, the storing unit 11 in each of the above-described embodiments may not be included in the pain measurement device 10 or 10A. In this case, the storing unit 11 may be a storing device connected to the pain measurement device 10 or 10A via a communication network.

Each of the above-described embodiments explains a case where the pain level is reported by a subject of measurement in VAS, but this does not necessarily need to be limited to VAS. Pain levels do not need to be explicitly reported by a subject of measurement. For example, when the action of a subject of measurement changes in accordance with the pain level, the action may be considered as reported pain levels.

The baseline stimulation amount in each of the above-described embodiments is one example, such that the amount is not limited thereto. For example, the baseline stimulation amount can be a mean value of the minimum value and the median value in the range 101.

Each of the above-described embodiments used a first characteristic value and a second characteristic value for comparing brainwave data of a subject with reference brainwave data, but these characteristic values do not need to be used. In such a case, brainwave data of a subject and reference brainwave data may be compared, for example, by pattern matching. Further, only one of the first and second characteristic values can be used.

The first characteristic value and the second characteristic valuevalue in each of the above-described embodiments are one example, such that the amounts are not limited thereto. For example, the first characteristic value may simply be a peak value, instead of a peak to peak value.

Further, the first evaluation value and the second evaluation value in each of the above-described embodiments are one example, such that the values are not limited thereto. For example, a linear distance or Mahalanobis distance may be used as the evaluation value.

Some or all of the constituent elements comprised by the pain measurement device in each of the above-described embodiments may be composed of a single system LSI (Large Scale Integration).

System LSIs are ultra-multifunctional LSIs manufactured by integrating multiple constituents on a single chip. Specifically, system LSIs are computer systems comprised of a microprocessor, ROM (Read Only Memory), RAM (Random Access Memory), and the like. A computer program is stored in the ROM. The microprocessor is operated in accordance with the computer program for the system LSI to accomplish the function thereof.

LSI is called a system LSI in this instance, but LSI may also be called an IC, LSI, super LSI, or ultra LSI depending on the difference in the degree of integration. Further, the approach of forming an integrated circuit is not limited to LSIs. This may be materialized with a dedicated circuit or all-purpose processor. After the manufacture of the LSI, a programmable FPGA (Field Programmable Gate Array) or a reconfigurable processor in which the connection or setting of a circuit cell inside an LSI can be reconfigured may be used.

Furthermore, if a technique of forming an integrated circuit that replaces LSI due to advancement in semiconductor technology or another derivative technique is developed, such a technique may certainly be used to integrate a function block. Application of biotechnology or the like is a possibility thereof.

In each of the above-described embodiments, each constituent element may be constructed with a dedicated hardware or materialized by executing a software program suited for each constituent element. Each constituent element may be materialized by a program executing unit, such as a CPU or processor, reading out and executing a software program recorded on a recording medium, such as a hard disk or a semiconductor memory. In this regard, software that materializes the pain measurement device of each of the above-described embodiments or the like includes the following programs.

Specifically, such a program makes a computer execute a pain measurement method for measuring pain being experienced by a subject of measurement, not part of the invention, comprising: a determination step for determining a baseline stimulation amount corresponding to baseline pain in the subject of measurement, based on a relationship between a pain level reported by the subject of measurement and a stimulation amount applied to the subject of measurement; and a measurement step for measuring pain being experienced by the subject of measurement, when brainwave data is measured by comparing brainwave data of a subject measured from the subject of measurement with reference brainwave data of the subject of measurement

[Industrial Applicability]

The present invention can be utilized as a pain measurement device for measuring pain being experienced by a subject of measurement.

[Reference Signs List]

- 10, 10A Pain measurement device
- 11 Storing unit
- 12 Determination unit
- 13 Measurement unit
- 14A Estimation unit
- 20 Stimulation device
- 30 Electroencephalograph

**Claims**

1. A pain measurement device (10) for measuring pain being experienced by a subject of measurement (99), comprising:

   a determination unit (12) for determining a baseline stimulation amount corresponding to baseline pain in the subject of measurement (99), based on a relationship between a pain level reported by the subject of measurement (99) and a stimulation amount applied to the subject of measurement (99); and
   a measurement unit (13) for measuring pain being experienced by the subject of measurement (99) when brainwave data of a subject is measured by comparing the brainwave data of a subject measured from the subject of measurement (99), with reference brainwave data of the subject of measurement (99), wherein the reference brainwave data is measured from the subject of measurement (99) when a stimulation at the baseline stimulation amount is applied to the subject of measurement (99) and obtained from a storing unit (11);
   **characterized in that** the determination unit (12) is configured to determine a representative value in a range (101) of stimulation amounts as said baseline stimulation amount, wherein said range (101) of stimulation amounts is a range where a ratio of increase in the pain level to an increase in the stimulation amount in a relationship between the pain level and the stimulation amount exceeds a predetermined threshold ratio.

2. The pain measurement device (1) of claim 1, wherein the pain measurement device (10) further comprises an estimation unit for estimating a second pain level to be reported by the subject of measurement (99) and second brainwave data to be measured from the subject of measurement (99), when a stimulation at a second stimulation amount that is different from a plurality of first stimulation amounts is applied to the subject of measurement (99), based on a first pain level reported by the subject of measurement (99) and first brainwave data measured from the subject of measurement (99) when stimulations at the plurality of first stimulation amount are individually applied to the subject of measurement (99);
   wherein the determination unit (12) determines the baseline stimulation amount based on a relationship between the first pain level and the second pain level, and the first stimulation amount and the second stimulation amount.

3. The pain measurement device (10) of claim 1 or claim 2, wherein the representative value is at least one of a minimum value, a median value, and a maximum value.

4. The pain measurement device (10) of any preceding claim, wherein the measurement unit (13) calculates at least one characteristic value from each of the brainwave data of a subject and the reference brainwave data, and

   measures pain being experienced by the subject of measurement (99), based on a result of comparison of at least one characteristic value calculated from the brainwave data of a subject, with at least one characteristic value calculated from the reference brainwave data.

5. The pain measurement device (10) of claim 4, wherein said at least one characteristic value comprises a first characteristic value representing an amplitude of a brainwave's waveform induced by a stimulation.


**Patentansprüche**

1. Schmerzmessvorrichtung (10) zum Messen von Schmerz, der durch einen Probanden der Messung (99) empfunden wird, die umfasst:

   eine Bestimmungseinheit (12) zum Bestimmen eines Grundlinienstimulationsbetrags, der einem Grundlinienschmerz in dem Probanden der Messung (99) entspricht, anhand einer Beziehung zwischen einem durch den Probanden der Messung (99) berichteten Schmerzniveau und einem auf den Probanden der Messung (99) angewandten Stimulationsbetrag; und
   eine Messeinheit (13) zum Messen von Schmerz, der durch den Probanden der Messung (99) empfunden wird, wenn Gehirnwellendaten eines Probanden durch Vergleichen der Gehirnwellendaten eines Probanden, die von dem Probanden der Messung (99) gemessen werden, mit Referenzgehirnwellendaten des Probanden der Messung (99) gemessen werden, wobei die Referenzgehirnwellendaten von dem Probanden der Messung (99) gemessen werden, wenn eine Stimulation mit dem Grundlinienstimulationsbetrag auf den Probanden der Messung (99) angewandt wird, und von einer Speichereinheit (11) erhalten werden;
   **dadurch gekennzeichnet, dass** die Bestimmungseinheit (12) konfiguriert ist, einen Repräsentationswert in einem Bereich (101) von Stimulationsbeträgen als den Grundlinienstimulationsbetrag zu bestimmen, wobei der Bereich (101) von Stimulationsbeträgen ein Bereich ist, in dem ein Verhältnis einer Zunahme des Schmerzniveaus zu einer Zunahme des Stimulationsbetrags in einer Beziehung zwischen dem Schmerzniveau und dem Stimulationsbetrag ein vorgegebenes Schwellenverhältnis übersteigt.

2. Schmerzmessvorrichtung (1) nach Anspruch 1, wobei die Schmerzmessvorrichtung (10) ferner eine Schätzeinheit zum Schätzen eines zweiten Schmerzniveaus, das durch den Probanden der Messung (99) zu berichten ist, und zweiter Gehirnwellendaten, die von dem Probanden der Messung (99) zu messen sind, wenn eine Stimulation mit einem zweiten Stimulationsbetrag, der von mehreren ersten Stimulationsbeträgen verschieden ist, auf den Probanden der Messung (99) angewandt wird, anhand eines ersten durch den Probanden der Messung (99) berichteten Schmerzniveaus und erster Gehirnwellendaten, die von dem Probanden der Messung (99) gemessen werden, wenn Stimulationen mit den mehreren ersten Stimulationsbeträgen individuell auf den Probanden der Messung (99) angewandt werden, umfasst;
   wobei die Bestimmungseinheit (12) den Grundlinienstimulationsbetrag anhand einer Beziehung zwischen dem ersten Schmerzniveau und dem zweiten Schmerzniveau und dem ersten Stimulationsbetrag und dem zweiten Stimulationsbetrag bestimmt.

3. Schmerzmessvorrichtung (10) nach Anspruch 1 oder Anspruch 2, wobei der Repräsentationswert ein Minimalwert und/oder ein Mittelwert und/oder ein Maximalwert ist.

4. Schmerzmessvorrichtung (10) nach einem vorhergehenden Anspruch, wobei die Messeinheit (13) mindestens einen kennzeichnenden Wert aus jeden Gehirnwellendaten eines Probanden und den Referenzgehirnwellendaten berechnet und
   einen Schmerz, der durch den Probanden der Messung (99) empfunden wird, anhand eines Ergebnisses eines Vergleichs mindestens eines kennzeichnenden Werts, der aus den Gehirnwellendaten eines Probanden berechnet wird, mit mindestens einem kennzeichnenden Wert, der aus den Referenzgehirnwellendaten berechnet wird, misst.

5. Schmerzmessvorrichtung (10) nach Anspruch 4, wobei der mindestens eine kennzeichnende Wert einen ersten kennzeichnenden Wert umfasst, der eine Größe einer Wellenform einer Gehirnwelle, die durch eine Stimulation induziert wird, repräsentiert.


**Revendications**

1. Dispositif de mesure de la douleur (10) destiné à mesurer la douleur ressentie par un sujet de mesure (99), comportant :

une unité de détermination (12) pour déterminer une quantité de stimulation de base correspondant à une douleur de base chez le sujet de mesure (99), sur la base d'une relation entre un niveau de douleur signalé par le sujet de mesure (99) et une quantité de stimulation appliquée au sujet de mesure (99) ; et
une unité de mesure (13) pour mesurer la douleur ressentie par le sujet de mesure (99) lorsque des données d'onde cérébrale d'un sujet sont mesurées en comparant les données d'onde cérébrale d'un sujet mesurées à partir du sujet de mesure (99), avec des données d'onde cérébrale de référence du sujet de mesure (99), dans lequel les données d'onde cérébrale de référence sont mesurées à partir du sujet de mesure (99) lorsqu'une stimulation à la quantité de stimulation de base est appliquée au sujet de mesure (99) et obtenue à partir d'une unité de stockage (11) ;
**caractérisé en ce que** l'unité de détermination (12) est configurée pour déterminer une valeur représentative dans une plage (101) de quantités de stimulation en tant que dite quantité de stimulation de base, dans lequel ladite plage (101) de quantités de stimulation est une plage où un rapport d'augmentation du niveau de douleur sur une augmentation de la quantité de stimulation dans une relation entre le niveau de douleur et la quantité de stimulation dépasse un rapport de seuil prédéterminé.

2. Dispositif de mesure de la douleur (1) selon la revendication 1, dans lequel le dispositif de mesure de la douleur (10) comporte en outre une unité d'estimation pour estimer un second niveau de douleur à signaler par le sujet de mesure (99) et des secondes données d'onde cérébrale à mesurer à partir du sujet de mesure (99), lorsqu'une stimulation à une seconde quantité de stimulation qui est différente d'une pluralité de premières quantités de stimulation est appliquée au sujet de mesure (99), sur la base d'un premier niveau de douleur signalé par le sujet de mesure (99) et de premières données d'onde cérébrale mesurées à partir du sujet de mesure (99) lorsque des stimulations à la pluralité de premières quantités de stimulation sont appliquées individuellement au sujet de mesure (99) ;
dans lequel l'unité de détermination (12) détermine la quantité de stimulation de base sur la base d'une relation entre le premier niveau de douleur et le second niveau de douleur, et entre la première quantité de stimulation et la seconde quantité de stimulation.

3. Dispositif de mesure de douleur (10) selon la revendication 1 ou la revendication 2, dans lequel la valeur représentative est au moins une valeur parmi une valeur minimale, une valeur médiane et une valeur maximale.

4. Dispositif de mesure de la douleur (10) selon l'une quelconque des revendications précédentes, dans lequel l'unité de mesure (13) calcule au moins une valeur caractéristique à partir de chacune des données d'onde cérébrale d'un sujet et des données d'onde cérébrale de référence, et
mesure la douleur ressentie par le sujet de mesure (99), sur la base d'un résultat de comparaison d'au moins une valeur caractéristique calculée à partir des données d'onde cérébrale d'un sujet, avec au moins une valeur caractéristique calculée à partir des données d'onde cérébrale de référence.

5. Dispositif de mesure de la douleur (10) selon la revendication 4, dans lequel ladite au moins une valeur caractéristique comporte une première valeur caractéristique représentant une amplitude d'une forme d'onde de l'onde cérébrale induite par une stimulation.

Fig. 1

Fig. 2

Start

S11 — Select stimulation amount

S12 — Apply stimulation

S13 — Measure brainwave data

S14 — Obtain pain level

S15 — Store stimulation amount, brainwave data and pain level

S16 — All stimulation amounts selected?

No

Yes

End

Fig. 3

```
              ┌──────────┐
              │  Start   │
              └──────────┘
                   │
                   ▼
S21   ┌────────────────────────────┐
      │  Determine baseline         │
      │  stimulation amount         │
      └────────────────────────────┘
                   │
                   ▼
S22   ┌────────────────────────────┐
      │  Obtain reference brainwave  │
      │  data                        │
      └────────────────────────────┘
                   │
                   ▼
S23   ┌────────────────────────────┐
      │  Calculate reference         │
      │  characteristic amount       │
      └────────────────────────────┘
                   │
                   ▼
S24   ┌────────────────────────────┐
      │  Obtain brainwave data of    │
      │  subject                     │
      └────────────────────────────┘
                   │
                   ▼
S25   ┌────────────────────────────┐
      │  Calculate characteristic    │
      │  amount of subject           │
      └────────────────────────────┘
                   │
                   ▼
S26   ┌────────────────────────────┐
      │  Compare characteristic      │
      │  amount of subject with      │
      │  reference characteristic    │
      │  amount                      │
      └────────────────────────────┘
                   │
                   ▼
S27   ┌────────────────────────────┐
      │  Measure pain based on result│
      │  of comparison               │
      └────────────────────────────┘
                   │
                   ▼
              ┌──────────┐
              │   End    │
              └──────────┘
```

Fig. 4

EP 3 263 026 B1

Fig. 5

Signal level · Time · N1 · N2 · T0 · P2 · P1 · T1

Fig. 6

S20A — Estimate pain level and brainwave data

S21A — Determine baseline stimulation amount

S22 — Obtain reference brainwave data

S23 — Calculate reference characteristic amount

S24 — Obtain brainwave data of subject

S25 — Calculate characteristic amount of subject

S26 — Compare characteristic amount of subject with reference characteristic amount

S27 — Measure pain based on result of comparison

Start

End

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080249430 A
- JP 2010523226 W